(19) Europäisches Patentamt / European Patent Office / Office européen des brevets

(11) **EP 3 976 978 B1**

(12) **FASCICULE DE BREVET EUROPEEN**

(45) Date de publication et mention de la délivrance du brevet:
**08.11.2023  Bulletin 2023/45**

(21) Numéro de dépôt: **20737242.6**

(22) Date de dépôt: **15.05.2020**

(51) Classification Internationale des Brevets (IPC):
**F16B 23/00** *(2006.01)*    **A61B 17/86** *(2006.01)*

(52) Classification Coopérative des Brevets (CPC):
**F16B 23/0092; A61B 17/8615; F16B 23/0023; F16B 23/003; F16B 23/0038;** A61B 17/8605

(86) Numéro de dépôt international:
**PCT/FR2020/050815**

(87) Numéro de publication internationale:
**WO 2020/240111 (03.12.2020 Gazette 2020/49)**

(54) **VIS POUR ANCRAGE OSSEUX DOTÉES D'UNE PLURALITÉ DE TYPE D'EMPREINTES**

KNOCHENVERANKERUNGSSCHRAUBEN MIT MEHREREN MUFFENTYPEN

BONE ANCHORING SCREWS PROVIDED WITH A PLURALITY OF SOCKET TYPES

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité:  **28.05.2019   FR 1905657**

(43) Date de publication de la demande:
**06.04.2022   Bulletin 2022/14**

(73) Titulaire: **Abys Medical**
**17000 La Rochelle (FR)**

(72) Inventeurs:
• **DESTAINVILLE, Arnaud**
**17138 SAINT-XANDRE (FR)**

• **RICHART, Olivier**
**17000 LA ROCHELLE (FR)**

(74) Mandataire: **IP Trust**
**2, rue de Clichy**
**75009 Paris (FR)**

(56) Documents cités:
EP-A2- 0 172 130        EP-A2- 2 278 175
US-A1- 2003 059 276   US-A1- 2006 266 168
US-A1- 2009 010 734   US-A1- 2009 220 321
US-A1- 2014 142 639   US-A1- 2017 238 982
US-A1- 2018 347 612   US-B1- 9 044 843

**Description**

## DOMAINE TECHNIQUE

La présente invention se rapporte à une vis dotée d'une pluralité de type d'empreintes pour son utilisation dans un contexte médical, en particulier dans celui de la chirurgie orthopédique, maxillo-faciale ou dentaire.

## ETAT DE LA TECHNIQUE ANTERIEURE

Par le terme vis, la présente description désigne, de manière non limitative, une vis comportant une tête de vis, éventuellement un fut, au moins une partie filetée et une pointe. La tête de vis reprend le couple exercé par un outil de serrage, par exemple un tournevis ou une clé, et applique un effort de plaquage.
Le fût peut être une partie cylindrique ou conique, lisse.
La partie filetée assure la mise en tension de la liaison par vissage au moyen d'une saillie hélicoïdale aussi désignée sous le nom de filet, laquelle est destinée à s'enfoncer en tournant dans le substrat.
La pointe assure le guidage de la vis, voire le perçage du substrat, par exemple dans du bois, du métal ou dans un écrou fileté, dans lequel est serrée la vis.
Les empreintes de vis sont variées et possèdent chacune leurs particularités, et permettent par conséquent des utilisations différentes. L'empreinte a un impact sur le couple de serrage, l'usure et l'efficacité du serrage. Il en existe plusieurs sortes, dont certaines sont très courantes, et d'autres un peu plus rares et moins utilisées. On peut noter :

- la vis à empreinte fendue ou plate qui présente une fente sur la tête de vis, permettant d'être fixée à l'aide d'un tournevis plat,
- la vis à empreinte cruciforme, aussi appelée vis Phillips, qui présente une petite croix sur la tête de vis. Elle s'utilise conjointement avec un tournevis cruciforme. On la reconnaît également par le marquage «PH» (pour Phillips),
- la vis à empreinte Pozidriv qui ressemble à la vis Phillips, du fait de la présence d'une petite croix. La vis Pozidriv possède en outre un croisillon repère qui la différencie de la vis Phillips,
- la vis à empreinte à six lobes internes, aussi appelée vis Torx, qui est reconnaissable grâce à sa forme en étoile,
- la vis à empreinte hexagonale creuse, également connue sous nom Allen,
- la vis à empreinte carrée, appelée aussi vis Robertson.

Les vis de fixation sont habituellement fabriquées à base d'acier. Lorsqu'une grande résistance au temps ou à la corrosion est requise, comme pour les petites vis de fixation ou implants médicaux, des matériaux tels que l'acier inoxydable ou le titane et les alliages de titane.
Du document EP0172130, on connaît une vis qui peut être entraînée par différentes tailles de tournevis de type Torx. Cette vis permet une plus grande souplesse quant à l'utilisation d'outils permettant de visser ces vis, dans le domaine particulier de la chirurgie orthopédique, ces vis étant destinées à être insérées dans un os pour permettre sa consolidation.
Toutefois, ce document ne divulgue la possibilité pour la tête de vis d'être entraînée par diverses formes de têtes, c'est-à-dire d'empreinte, de tournevis.
Du document WO2003025403, on connaît une vis comportant un évidement présentant trois étages superposés non circulaires, le premier étage présentant une empreinte hexagonale creuse d'un premier diamètre, le deuxième étage présentant une empreinte hexagonale creuse d'un deuxième diamètre inférieur au premier diamètre, laquelle est tournée angulairement d'un angle de 30° par rapport à la première empreinte selon un axe perpendiculaire au plan principal de l'empreinte, et un troisième étage à empreinte pentagonale d'un troisième diamètre inférieur au deuxième diamètre.
Par diamètre d'un étage s'étendant perpendiculairement à un axe, la présente description vise la plus grande dimension de l'évidement dans un plan perpendiculaire audit axe. Dans le cas d'un évidement formé par un cylindre à base hexagonal, le diamètre de l'évidement est le double du rayon du cercle inscrit. De nombreux types d'empreintes de tête de vis peuvent être utilisées dans le domaine de la chirurgie osseuse. La réglementation médicale impose d'analyser tous les risques inhérents à l'utilisation de matériels implantables, et de mettre en oeuvre toutes les actions possibles pour en minimiser les impacts en termes de performance et de sécurité pour le patient. Dans cette optique le besoin de permettre une extraction du matériel - quelles qu'en soient les raisons ou les circonstances - est une nécessité. Il existe donc un besoin de pouvoir assurer que la majorité des tournevis utilisés en standard en chirurgie pourra coopérer avec les empreintes les plus couramment utilisées dans le but de l'extraire du corps du patient.
On connaît les documents suivants se rapportant à des vis chirurgicales: EP0172130 A2, US2017/238982 A1, US2014/142639 A1, US 9044843 B1, US 2006/266168 A1.
Il existe un besoin d'améliorer encore la souplesse d'utilisation de différents outils permettant de visser ces vis, mais surtout de les dévisser et de pouvoir satisfaire l'obligation réglementaire précitée.

## EXPOSE DE L'INVENTION

Un but de l'invention est notamment de remédier à tout ou partie des inconvénients précités.

À cet effet, et comme défini dans la revendication 1, il est proposé une vis pour ancrage osseux comportant une tête, un fut, par exemple cylindrique, et une partie filetée disposés successivement le long d'un axe commun, la tête présentant un évidement comportant au moins deux étages disposés successivement le long de l'axe commun, le premier étage présentant un diamètre supérieur au diamètre du deuxième étage. Selon l'invention, l'un des deux étages comporte au moins une empreinte d'un premier type et l'autre des deux étages comporte au moins une empreinte du premier type et d'un autre type.

Par exemple, l'empreinte du premier type et l'autre empreinte peuvent être, respectivement, à six lobes (selon la norme ISO 10664) ou à six pans creux (selon la norme ISO 4762 ou ISO 10642). Toujours selon l'invention , la vis comporte en outre un étage intermédiaire disposé entre le premier et le deuxième étage et l'évidement de l'étage intermédiaire comporte moins de types d'empreintes que l'évidement du premier et/ou du deuxième étage.

Selon une possibilité, un étage peut s'étendre au moins entre une surface transverse (éventuellement une couronne) disposée du côté du fût formant un épaulement (tourné vers la tête sur lequel peut s'appuyer un tournevis) et une couronne transverse disposée du côté de la tête (c'est-à-dire axialement du côté opposé au fût), l'étage présentant un évidement formé de l'union (au sens géométrique) d'une empreinte à six lobes et d'une empreinte à six pans creux, chacune des empreintes étant centrée sur l'axe commun et s'ouvrant du côté de la tête. Aussi, les plans transverses médians de chacune des empreintes peuvent être confondus.

Avantageusement, l'étage peut présenter, dans une coupe transverse à l'axe commun, un contour présentant successivement en alternance un sommet de l'empreinte à six pans creux et un sommet d'une empreinte à six lobes.

Un étage peut être un cylindre dont la base a pour contour celui résultant d'un contour six lobes et d'un contour six pans et se terminant par un épaulement transverse.

Selon un mode de réalisation particulier, l'angle formé entre deux sommets successifs est sensiblement égal 30° autour de l'axe commun.

De manière plus précise, l'empreinte à six lobes peut présenter un rayon $r_{6lc}$ de cercle circonscrit et un rayon $r_{6li}$ de cercle inscrit et l'empreinte à six pans creux peut présenter un rayon $r_{6pc}$ de cercle circonscrit et un rayon $r_{6pi}$ de cercle inscrit, et les rayons vérifient l'inégalité suivante :

[Math.1]

$$r_{6l\,i} < r_{6p\,i} = \frac{\sqrt{3}}{2}\, r_{6p\,c} < r_{6l\,c}.$$

L'évidement de l'étage peut en outre comporter l'union (au sens géométrique) d'une empreinte cruciforme de type Z et d'une empreinte cruciforme de type H.

Les quatre petites branches de l'empreinte cruciforme de type Z peuvent être contenues dans les quatre branches de l'empreinte cruciforme de type H (par contenu dans, on entend s'inscrivent dans l'évidement formé par). De préférence, le diamètre de l'empreinte de type H peut être supérieur au diamètre de l'empreinte à six pans creux. Un sommet d'une grande branche d'une empreinte de type Z, le centre de ladite empreinte, et un sommet d'une empreinte à six pans creux peuvent être alignés.

Selon une possibilité, les deux étages peuvent être identiques ou l'étage comportant au moins l'empreinte du premier type peut être conforme à l'autre étage précédemment décrit.

Les empreintes de type Z et/ou H et/ou carré et/ou fente peuvent être obtenues par homothétie entre deux étages. Les empreintes de type six pans et/ou six lobes peuvent être obtenues par homothétie et rotation entre deux étages.

## DESCRIPTION DES FIGURES

D'autres avantages et particularités de l'invention apparaîtront à la lecture de la description détaillée de mises en oeuvre et de modes de réalisation nullement limitatifs, au regard de dessins annexés sur lesquels :

La figure 1 illustre dans sa partie supérieure une vue en perspective avec arraché d'un premier exemple de vis; la partie inférieure est une coupe longitudinale de la vis selon lignes AA de la partie supérieure;

La figure 2 présente en partie gauche une coupe selon lignes DD de la partie droite; la partie droite représente une mise en plan de la partie supérieure de la figure 1;

La figure 3 comporte une partie supérieure présentant en partie gauche une coupe selon lignes EE de la partie droite, la partie droite représentant une mise en plan d'une vis à trois étages, chacun des étages comportant une empreinte à six lobes selon norme ISO 10 664 ; la partie inférieure présente en partie gauche une coupe selon lignes FF de la partie droite, la partie droite représentant une mise en plan d'une vis à trois étages, chacun des étages comportant une empreinte à six pans creux;

La figure 4 présente en partie gauche une coupe selon lignes DD de la partie droite; la partie droite représente une mise en plan d'une vis selon un deuxième exemple;

La figure 5 présente en partie gauche une coupe selon lignes HH de la partie droite, la partie droite représentant une mise en plan d'une vis à trois étages, chacun des étages comportant une empreinte cruciforme de type H selon norme ISO 4757;

La figure 6 présente en partie gauche une coupe selon lignes GG de la partie droite, la partie droite représentant une mise en plan d'une vis à trois étages, chacun des étages comportant une empreinte cruciforme de type Z selon norme ISO 4757;

La figure 7 présente en partie gauche une coupe selon lignes DD de la partie droite; la partie droite représente une mise en plan d'une vis selon un troisième exemple;

La figure 8 présente en partie gauche une coupe selon lignes II de la partie droite, la partie droite représentant une mise en plan d'une vis à trois étages, le premier et le deuxième étage comportant une vis carrée.

## DESCRIPTION DE MODES DE REALISATION

Bien qu'elle ne soit pas limitée aux vis à usage orthopédique, la présente invention trouve une utilité particulière dans ce domaine.

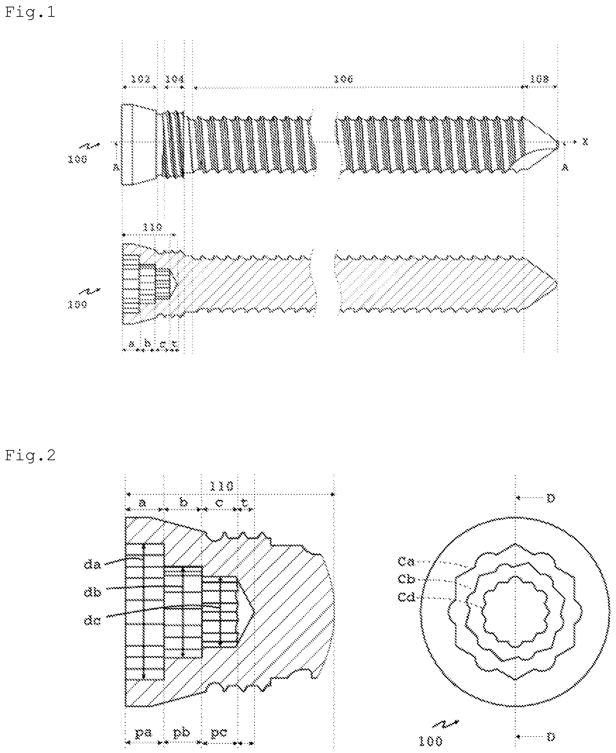

En référence à la partie supérieure de la figure 1, il est décrit un premier exemple de vis 100.

La vis 100 présente un axe longitudinal X et comporte successivement, selon une direction de l'axe longitudinal, une tête 102, une première partie filetée 104, une deuxième partie filetée 106 et une pointe 108.

Une première partie de transition (non numérotée), aussi appelée première partie de fût, est disposée axialement entre la partie de tête 102 et la première partie filetée 104.

Une deuxième partie de transition (non numérotée), aussi appelée deuxième partie de fût, est disposée axialement entre la première partie filetée 104 et la deuxième partie filetée 106.

En référence à la partie inférieure de la figure 1, on observe que la vis comporte, du côté de sa tête 102, un évidement 110 comportant trois étages disposés successivement selon la direction précitée de l'axe longitudinal X, respectivement référencés a, b, c sur la figure mais 102a, 102b et 102c dans la présente description. L'évidement 110 comporte en outre une fraisure de terminaison, référencée t sur la figure, mais 102t dans la présente description.

Dans cet exemple, l'extension axiale de l'évidement 110 est de 4,37 mm et le demi-angle au sommet du cône formé par fraisure est de 62°.

La profondeur de l'évidement 110 peut avantageusement être supérieure à la profondeur de la tête 102, de préférence supérieure à la profondeur de la tête 102 et de la première partie de transition, de manière plus préférée supérieure à la profondeur de la tête 102 et de la première partie de transition et une fraction de la profondeur de la première partie filetée 104. Cette caractéristique permet d'optimiser la compacité de la vis.

Chacun des étages présente un diamètre inférieur à l'étage immédiatement voisin du côté de la tête 102 et supérieur au diamètre de l'étage immédiatement voisin du côté de la pointe 108.

Le premier étage 102a présente un diamètre inférieur au diamètre extérieur de la vis 100.

Le cône 102t présente un diamètre inférieur au diamètre du dernier étage.

La suite des diamètres, respectivement da, db, de des étages 102a, 102b, 102c est une suite décroissante.

En référence à la partie de gauche de la figure 2, il est à noter que la suite des profondeurs, respectivement pa, pb, pc des étages 102a, 102b, 102c peut être une suite décroissante. Alternativement, la suite des profondeurs des étages peut être constante, croissante ou quelconque.

Les contours, respectivement, Ca, Cb et Cd, des étages avec 102a, 102b et 102c sont représentés.

Chacun des contours résulte de l'union de deux surfaces, l'une des surfaces résultant d'une empreinte à six lobes selon norme ISO 10664 et l'autre des surfaces résultant d'une empreinte à six pans creux.

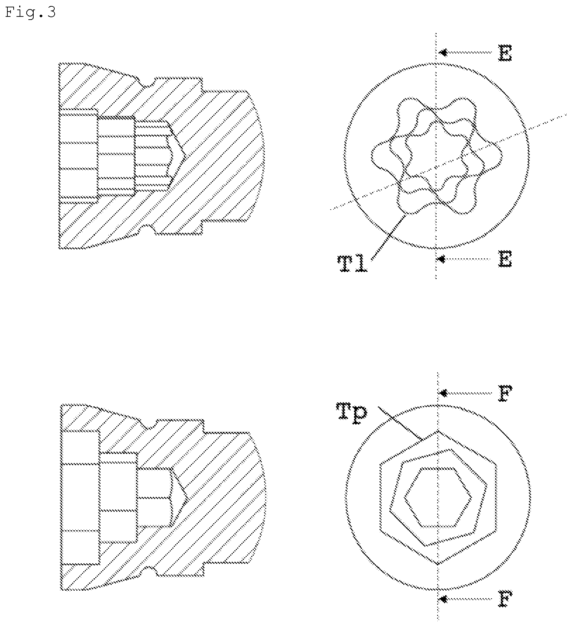

Pour la bonne compréhension de la divulgation, la figure 3 représente deux vis.

En référence à la partie supérieure de la figure 3 :

- les dimensions des premier, deuxième, et troisième étages sont conformes, respectivement, aux dimensions T25, T15 et T8 de la norme ISO 10664, c'est-à-dire des diamètres intérieurs respectifs de 3,25 mm, 2,40 mm et 1,75 mm (cercles inscrits de rayon respectif 1,652 mm, 1,20 mm et 0,875 mm),
- le deuxième étage est angulairement tourné de 45° par rapport au premier étage,
- le troisième étage est angulairement tourné de 45° par rapport au deuxième étage.

En référence à la partie inférieure de la figure 3 :

- les dimensions des premier, deuxième, et troisième étages sont conformes, respectivement, aux dimensions M5, M4, M2,5 de la norme la norme ISO 4762, c'est-à-dire que la distance entre deux faces opposées respectives est de 4mm, 3mm et 2mm (cercles inscrits de rayon respectif, 2 mm, 1,5 mm et 1 mm et cercles circonscrits de rayon),
- le deuxième étage est angulairement tourné de 45° par rapport au premier étage,
- le troisième étage est angulairement tourné de 45° par rapport au deuxième étage.

Aussi, dans l'exemple représenté sur la figure 2 :

- chacun des étages présente une profondeur de 1,30 mm,
- chacun des étages est tourné angulairement de 45° autour de l'axe commun par rapport à l'étage précédent/suivant,
- la fraisure présente une dimension de 2,47 mm.

L'évidement 110 est le résultat de l'union des deux vis à trois étages qui viennent d'être décrites.

Chacun des contours présente successivement en alternance, un sommet d'une empreinte à six pans creux et un sommet d'une empreinte à six lobes. L'angle formé entre deux sommets consécutifs est de 30°, de sorte d'obtenir une invariante de contours par rotation de 60°.

Pour chacun des étages, les plans transverses médians de l'empreinte à six lobes et de l'empreinte à six pans sont confondus.

Lorsqu'une empreinte à six lobes est tournée centrée sur une empreinte à six pans creux et tournée angulairement de sorte que le contour résultant présente en alternance un sommet d'une empreinte à six pans creux et un sommet d'une empreinte à six lobes, il est nécessaire que :

- le rayon r6lc du cercle circonscrit des empreintes à six lobes soit plus grand que le rayon r6pi du cercle inscrit (aussi désigné sous le terme apothème) des empreintes à six pans creux : r6lc > r6pi,
- le rayon r6pc du cercle circonscrit des empreintes à six pans creux, soit plus grand que le rayon r6li du cercle inscrit des empreintes à six lobes, r6pc > r6li,
- le rayon r6pi du cercle inscrit des empreintes à six pans creux soit plus grand que le rayon r6li du cercle inscrit des empreintes à six lobes : r6pi > r6li.

Ces conditions peuvent être résumées par l'inégalité suivante :

[Math. 1]

$$r_{6l\,i} < r_{6p\,i} = \frac{\sqrt{3}}{2}\, r_{6p\,c} < r_{6l\,c}$$

Ces conditions sont bien vérifiées pour chacun des trois étages, comme le montre le tableau qui suit :

[Tableau 1]

|  |  | $r_{6li}$ | $r_{6pi}$ | $r_{6pc}$ | $r_{6lc}$ |
|---|---|---|---|---|---|
| Etage 1 | T25 et M5 | 1 625 | 2 000 | 2 309 | 2 250 |
| Etage 2 | T15 et M4 | 1 200 | 1 500 | 1 732 | 1 675 |
| Etage 3 | T8 et M2, 5 | 0,875 | 1 000 | 1 155 | 1 200 |

La transformation géométrique qui transforme le premier contour Ca en le deuxième contour Cb est une similitude d'angle 45°. La transformation géométrique qui transforme le deuxième contour Cb en le troisième contour Cd est également une similitude d'angle 45°.

Une telle vis peut être formée par usinage ou par fabrication additive.

Le matériau utilisé pour former la vis peut être un matériau ayant des propriétés antibactériennes.

Selon une première variante, non représentée, uniquement décrite pour ses différences avec le premier mode de réalisation, la vis peut ne comporter que deux étages au lieu de trois.

Selon une deuxième variante, non représentée, uniquement décrite pour ses différences avec la première variante, l'un des étages présente un contour formé de deux types distincts d'empreintes de diamètre sensiblement identique, l'autre des étages comportant une seule empreinte, d'un même type que l'un des types formant ledit contour.

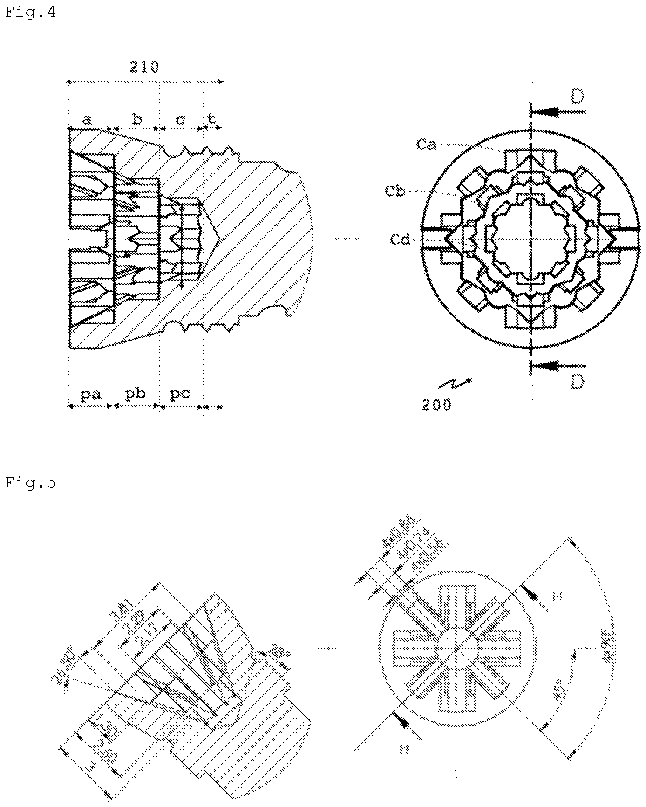

En référence à la figure 4, il est représenté une vis 200 selon un deuxième exemple.

La vis 200 comporte un évidement 210 encore formé de trois étages a, b, c, référencés 210a, 210b, 210c dans la description.

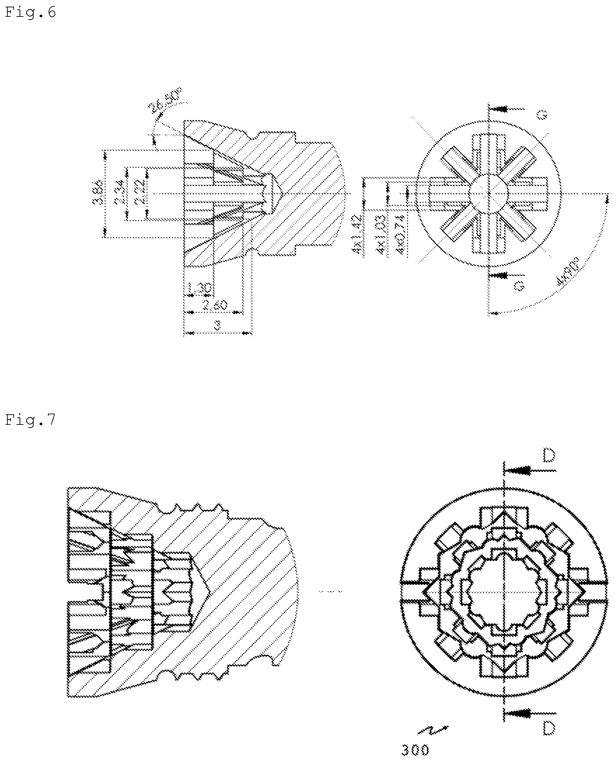

Comme cela sera mieux compris à la lumière de la description des figures 5 et 6, chacun des étages est formé :

- d'une empreinte à six lobes selon norme ISO 10664,
- d'une empreinte à six pans creux,
- d'une empreinte cruciforme de type Z (type Pozidriv),
- d'une empreinte cruciforme de type H (type Phillips).

Une empreinte cruciforme de type H comporte quatre branches formant une croix, chacune des branches ayant les mêmes dimensions.

Une empreinte cruciforme de type Z présente une forme d'étoile à 8 branches : quatre grandes branches comme celles des empreintes H, et quatre petites branches de mêmes dimensions et formant une croix tournée de 45° par rapport à la croix formée par les quatre grandes branches. La dimension de chacune des quatre petites branches est inférieure à celle des grandes branches.

Comme visible sur la figure 5, les différentes empreintes cruciformes de type H de chacun des étages sont centrées entre elles et ne sont pas tournées les unes par rapport aux autres selon l'axe commun entre deux étages successifs.

Toujours en référence à la figure 5 :

- le premier étage comporte une empreinte cruciforme de type H de taille PH3 conforme à la norme ISO 4757, chacune des branches ayant la même dimension, le diamètre de l'empreinte étant de 3,81 mm,
- le deuxième étage comporte une empreinte cruciforme de type H de taille PH2 conforme à la norme ISO 4757, chacune des branches ayant la même dimension, le diamètre de l'empreinte étant de 2,29 mm,
- le troisième étage comporte une empreinte cruciforme de type H de taille PH1 conforme à la norme ISO 4757, chacune des branches ayant la même dimension, le diamètre de l'empreinte étant de 2,22 mm.

En référence à la figure 6 :

- le premier étage comporte une empreinte cruciforme de type Z de taille PZ3 conforme à la norme ISO 4757, les grandes branches présentant un diamètre de 3,86 mm, les petites branches présentant un diamètre de 3,81 mm,
- le deuxième étage comporte une empreinte cruciforme de type H de taille PZ2 conforme à la norme ISO 4757, les grandes branches présentant un diamètre de 2,34 mm, les petites branches présentant un diamètre de 2,29 mm,
- le troisième étage comporte une empreinte cruciforme de type H de taille PZ1 conforme à la norme ISO 4757, les grandes branches présentant un diamètre de 2,22 mm, les petites branches présentant un diamètre de 2,17 mm.

Comme visible sur la figure 6, sur chaque étage, les quatre branches de l'empreinte cruciforme de type H sont tournées de 45° autour de l'axe commun par rapport aux quatre grandes branches de l'empreinte cruciforme de type Z.

De manière astucieuse, les branches de petit diamètre d'une empreinte de type Z d'un étage présentent une extension compatible avec (donc supérieure à) celle d'une empreinte cruciforme de type H pour le même étage.

En référence à la figure 4, et plus particulièrement à la figure de gauche, on note que, pour chacun des trois étages, le plan transverse médian de l'empreinte cruciforme de type Z est confondu avec chacun des plans transverses médians de l'empreinte à six lobes et de l'empreinte à six pans creux.

Selon une première variante du deuxième exemple, non représentée, uniquement décrite pour ses différences avec le deuxième exemple, la vis peut ne comporter que deux étages au lieu de trois.

Selon une deuxième variante du deuxième exemple, non représentée, uniquement décrite pour ses différences avec le deuxième exemple, et éventuellement combinable avec la première variante du deuxième exemple, ou le premier exemple ou l'une, ou plusieurs, de ses variantes, l'un des étages ne comporte que les (ou encore une seule des) deux empreintes cruciformes de type H et/ou Z.

Selon une troisième variante du deuxième exemple, non représentée, uniquement décrite pour ses différences avec le premier et/ou le deuxième exemple et éventuellement combinable avec la première variante, ou le premier exemple ou l'une de ses variantes, l'un des étages ne comporte que les (ou encore une seule des) deux empreintes de type à six lobes et/ou à six pans creux.

En référence à la figure 7, il est représenté une vis 300 selon un troisième exemple.

Chacun des étages est formé :

- d'une empreinte à six lobes selon norme ISO 10664,

- d'une empreinte à six pans creux,
- d'une empreinte cruciforme de type Z (type Pozidriv),
- d'une empreinte cruciforme de type H (type Phillips).

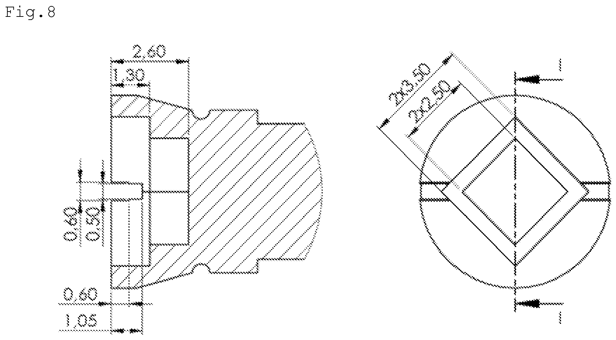

Comme cela sera mieux compris à la lumière de la description des figures 8 :

- le premier étage et le deuxième étage comportent chacun une empreinte carrée,
- le premier étage comporte deux empreintes fentes.

Comme visible sur la figure 8, les différentes empreintes carrées étages sont centrées entre elles et ne sont pas tournées les unes par rapport aux autres selon l'axe commun entre deux étages successifs.
Toujours en référence à la figure 8 :

- le premier étage comporte une empreinte carrée de côté 3,50, présentant un diamètre de 4,95 mm,
- le deuxième étage comporte une empreinte carrée de côté 2,50, présentant un diamètre de 3,54 mm.

Le premier étage comporte en outre deux empreintes fentes. Chacune des empreintes feintes présente un plan d'extension qui est transverse par rapport à l'axe commun. Les deux plans d'extensions sont confondus, et perpendiculaires au plan axial passant par deux sommets opposés d'un des carrés.
En référence à la figure 7, et plus particulièrement à la figure de gauche, on note que, pour chacun des deux premiers étages, le plan transverse médian de l'empreinte carré est confondu avec chacun des plans transverses médians de l'empreinte à six lobes et de l'empreinte à six pans creux.

## Revendications

1. Vis pour ancrage osseux comportant une tête (102), un fut et une partie filetée (106) disposés successivement le long d'un axe commun (X), la tête présentant un évidement (110) comportant au moins deux étages (a, b, c) disposés successivement le long de l'axe commun, le premier étage présentant un diamètre (da) supérieur au diamètre du deuxième étage (db),

   **caractérisée en ce que** l'un des deux étages comporte au moins une empreinte d'un premier type (Tl; Tp) et l'autre des deux étages comporte au moins une empreinte dudit premier type et d'un autre type (Tp; Tl), comportant en outre un étage intermédiaire disposé entre le premier et le deuxième étage, dans laquelle l'évidement de l'étage intermédiaire comporte moins de types d'empreintes que l'évidement du premier et/ou du deuxième étage.

2. Vis selon la revendication précédente, dans laquelle dans l'autre étage, l'empreinte du premier type et l'autre empreinte sont, respectivement, à six lobes (Tl) et à six pans creux (Tp) ou l'empreinte du premier type et l'autre empreinte sont, respectivement, à six pans creux (Tp) et à six lobes (Tl).

3. Vis selon la revendication précédente, dans laquelle un étage s'étend au moins entre une surface transverse disposée du côté du fût formant un épaulement et une couronne transverse disposée du côté de la tête, l'étage présentant un évidement formé de l'union d'une empreinte à six lobes et d'une empreinte à six pans creux, chacune des empreintes étant centrée sur l'axe commun et s'ouvrant du côté de la tête.

4. Vis selon la revendication précédente, dans laquelle l'étage présente, dans une coupe transverse à l'axe commun, un contour présentant successivement en alternance un sommet de l'empreinte à six pans creux et un sommet d'une empreinte à six lobes.

5. Vis selon la revendication précédente, dans laquelle l'angle formé entre deux sommets successifs est sensiblement égal 30° autour de l'axe commun.

6. Vis selon la revendication 4 ou la revendication précédente, dans laquelle l'empreinte à six lobes présente un rayon $r_{6lc}$ de cercle circonscrit et l'empreinte à six pans creux présente un rayon $r_{6pc}$ de cercle circonscrit et un rayon $r_{6pi}$ de cercle inscrit, et les rayons vérifient l'inégalité suivante :

$$r_{6p\,i} = \frac{\sqrt{3}}{2}\, r_{6p\,c} < r_{6l\,c}$$

**7.** Vis selon l'une quelconque des revendications 3 à 6, dans laquelle l'évidement de l'étage comporte en outre l'union d'une empreinte cruciforme de type Z et d'une empreinte cruciforme de type H.

**8.** Vis selon la revendication précédente, dans laquelle les quatre petites branches de l'empreinte cruciforme de type Z sont contenues dans les quatre branches de l'empreinte cruciforme de type H.

**9.** Vis selon la revendication 7 ou la revendication précédente, dans laquelle le diamètre de l'empreinte de type H est supérieur au diamètre de l'empreinte à six pans creux.

**10.** Vis selon l'une quelconque des revendications 7 à 9, dans laquelle un sommet d'une grande branche d'une empreinte de type Z, le centre de ladite empreinte, et un sommet d'une empreinte à six pans creux sont alignés.

**11.** Vis selon l'une quelconque des revendications précédentes, dans laquelle les types d'empreintes des deux étages sont identiques ou l'étage comportant au moins l'empreinte du premier type est conforme à l'autre étage qui est décrit en référence à l'une quelconque des revendications 2 à 10.

**12.** Vis selon l'une quelconque des revendications précédentes, dans laquelle l'une au moins des empreintes d'un étage peut être obtenue par homothétie de l'une au moins des empreintes de l'autre étage ou obtenue par homothétie et rotation de l'une au moins des empreintes de l'autre étage.

## Patentansprüche

**1.** Knochenverankerungsschraube, die einen Kopf (102), einen Schaft und einen Gewindeteil (106) aufweist, die aufeinanderfolgend entlang einer gemeinsamen Achse (X) angeordnet sind, wobei der Kopf eine Aussparung (110) vorweist, die mindestens zwei Stufen (a, b, c) aufweist, die aufeinanderfolgend entlang der gemeinsamen Achse angeordnet sind, wobei die erste Stufe einen Durchmesser (da) vorweist, der größer als der Durchmesser der zweiten Stufe (db) ist,

dadurch gekennzeichnet, dass die eine der zwei Stufen mindestens eine Vertiefung einer ersten Art (TI; Tp) aufweist und die andere der zwei Stufen mindestens einen Vertiefung der ersten Art und einer anderen Art (Tp; TI) aufweist,
die ferner eine Zwischenstufe aufweist, die zwischen der ersten und der zweiten Stufe angeordnet ist, wobei die Aussparung der Zwischenstufe weniger Vertiefungsarten als die Aussparung der ersten und/oder der zweiten Stufe aufweist.

**2.** Schraube nach dem vorstehenden Anspruch, wobei in der anderen Stufe die Vertiefung der ersten Art und die andere Vertiefung jeweils mit Innensechsrund (TI) und mit Innensechskant (Tp) sind oder die Vertiefung der ersten Art und die andere Vertiefung jeweils mit Innensechskant (Tp) und mit Innensechsrund (TI) sind.

**3.** Schraube nach dem vorstehenden Anspruch, wobei sich eine Stufe mindestens zwischen einer Queroberfläche, die auf der Seite des Schafts angeordnet ist, die einen Absatz ausbildet, und einer Querkrone erstreckt, die auf der Seite des Kopfs angeordnet ist, wobei die Stufe eine Aussparung vorweist, die aus der Vereinigung einer Innensechsrundvertiefung und einer Innensechskantvertiefung ausgebildet ist, wobei jede der Vertiefungen auf der gemeinsamen Achse zentriert ist und sich auf der Seite des Kopfes öffnet.

**4.** Schraube nach dem vorstehenden Anspruch, wobei die Stufe in einem Querschnitt quer zu der gemeinsamen Achse einen Umriss vorweist, der aufeinanderfolgend abwechselnd einen Scheitelpunkt der Innensechskantvertiefung und einen Scheitelpunkt einer Innensechsrundvertiefung vorweist.

**5.** Schraube nach dem vorstehenden Anspruch, wobei der Winkel, der zwischen zwei aufeinanderfolgenden Scheitelpunkten ausgebildet ist, im Wesentlichen gleich 30° um die gemeinsame Achse herum ist.

**6.** Schraube nach Anspruch 4 oder dem vorstehenden Anspruch, wobei die Innensechsrundvertiefung einen Umkreisradius $r_{6lc}$ vorweist und die Innensechskantvertiefung einen Umkreisradius $r_{6pc}$ und einen Inkreisradius $r_{6pi}$ vorweist und die Radien die folgende Ungleichung erfüllen:

$$r_{6pi} = \frac{\sqrt{3}}{2} r_{6pc} < r_{6lc}$$

**7.** Schraube nach einem der Ansprüche 3 bis 6, wobei die Aussparung der Stufe ferner die Vereinigung einer Kreuzschlitzschraubenvertiefung einer Art Z und einer Kreuzschlitzschraubenvertiefung einer Art H aufweist.

**8.** Schraube nach dem vorstehenden Anspruch, wobei die vier kleinen Schenkel der Kreuzschlitzschraubenvertiefung der Art Z in den vier Schenkeln der Kreuzschlitzschraubenvertiefung der Art H enthalten sind.

**9.** Schraube nach Anspruch 7 oder dem vorstehenden Anspruch, wobei der Durchmesser der Vertiefung der Art H größer als der Durchmesser der Innensechskantvertiefung ist.

**10.** Schraube nach einem der Ansprüche 7 bis 9, wobei ein Scheitelpunkt eines großen Schenkels einer Vertiefung der Art Z, das Zentrum der Vertiefung und ein Scheitelpunkt einer Innensechskantvertiefung aneinander ausgerichtet sind.

**11.** Schraube nach einem der vorstehenden Ansprüche, wobei die Arten der Vertiefungen der zwei Stufen identisch sind oder die Stufe, die mindestens die Vertiefung der ersten Art aufweist, mit der anderen Stufe übereinstimmt, die unter Bezugnahme auf einen der Ansprüche 2 bis 10 beschrieben ist.

**12.** Schraube nach einem der vorstehenden Ansprüche, wobei die mindestens eine der Vertiefungen einer Stufe durch Homothetie der mindestens einen der Vertiefungen der anderen Stufe erhalten werden kann oder durch Homothetie und Drehung der mindestens einen der Vertiefungen der anderen Stufe erhalten werden kann.

## Claims

**1.** Bone anchoring screw comprising a head (102), a shaft and a threaded portion (106) arranged successively along a common axis (X), the head having a recess (110) comprising at least two stages (a, b, c) arranged in succession along the common axis, the first stage having a diameter (da) larger than the diameter (db) of the second stage,

**characterized in that** one of the two stages comprises at least one socket of a first type (Tl; Tp) and the other of the two stages comprises at least one socket of the first type and one other type (Tp; Tl), the screw further comprising an intermediate stage arranged between the first and the second stage, the recess of the intermediate stage comprising fewer types of sockets than the recess of the first and/or second stage.

**2.** Screw according to the preceding claim, wherein, in the other stage, the socket of the first type and the other socket are, respectively, a hexalobular socket (Tl) and a hexagon socket (Tp), or the socket of the first type and the other socket are, respectively, a hexagon socket (Tp) and a hexalobular socket (Tl).

**3.** Screw according to the preceding claim, wherein a stage extends at least between a transverse surface arranged on the side of the shaft forming a shoulder and a transverse crown arranged on the side of the head, the stage having a recess formed by the union of a hexalobular socket and a hexagon socket, each of the sockets being centered on the common axis and opening on the side of the head.

**4.** Screw according to the preceding claim, wherein the stage has, in a section transverse to the common axis, a contour successively having, in alternation, a vertex of the hexagon socket and a vertex of a hexalobular socket.

**5.** Screw according to the preceding claim, wherein the angle formed between two successive vertices is substantially equal to 30° around the common axis.

**6.** Screw according to claim 4 or the preceding claim, wherein the hexalobular socket has a radius $r_{6lc}$ of a circumscribed circle and the hexagon socket has a radius $r_{6pc}$ of a circumscribed circle and a radius $r_{6pi}$ of an inscribed circle, and the radii satisfy the following inequality:

$$r_{6p\,i} = \frac{\sqrt{3}}{2}\, r_{6p\,c} < r_{6l\,c}$$

7.  Screw according to one of claims 3 to 6, wherein the recess of the stage further comprises the union of a Z-type crosshead socket and an H-type crosshead socket.

8.  Screw according to the preceding claim, wherein the four small branches of the Z-type crosshead socket are contained in the four branches of the H-type crosshead socket.

9.  Screw according to claim 7 or the preceding claim, wherein the diameter of the H-type socket is larger than the diameter of the hexagon socket.

10. Screw according to one of claims 7 to 9, wherein a vertex of a large branch of a Z-type socket, the center of said socket and a vertex of a hexagon socket are aligned.

11. Screw according to one of the preceding claims, wherein the types of sockets of the two stages are the same or the stage comprising at least the socket of the first type conforms to the other stage that is described with reference to one of claims 2 to 10.

12. Screw according to one of the preceding claims, wherein at least one of the sockets of one stage can be obtained by homothety of at least one of the sockets of the other stage or obtained by homothety and rotation of at least one of the sockets of the other stage.

Fig.1

Fig.2

Fig.3

Fig.4

Fig.5

Fig.6

Fig.7

Fig.8

**EP 3 976 978 B1**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 0172130 A
- WO 2003025403 A
- EP 0172130 A2
- US 2017238982 A1
- US 2014142639 A1
- US 9044843 B1
- US 2006266168 A1